# FASCICULE DE BREVET EUROPEEN

(11) **EP 1 439 909 B1**
(45) Date de publication et mention de la délivrance du brevet: **13.07.2005**
(21) Numéro de dépôt: 02795349.6
(22) Date de dépôt: 28.10.2002
(51) Int. Cl.: B01J 35/00, B01J 23/30, B01J 27/224

(54) **PHOTOCATALYSEUR ET PROCEDE DE PURIFICATION D'EFFLUENTS GAZEUX**
PHOTOKATALYSATOR UND ABGASREINIGUNGSVERFAHREN
PHOTOCATALYST AND A METHOD OF PURIFYING GASEOUS EFFLUENTS

(30) Priorité: 29.10.2001 FR 0113984
(43) Date de publication de la demande: 28.07.2004
(73) Titulaire: SICAT, 75008 Paris (FR); CENTRE NATIONAL DE LA RECHERCHE SCIENTIFIQUE (CNRS), 75794 Paris Cedex 16 (FR); Université Louis Pasteur de Strasbourg, F-67000 Strasbourg (FR)
(72) Inventeur: SPITZER-KELLER, Valérie, F-67200 Strasbourg (FR); BERNHARDT, Pierre, F-67190 Heiligenberg (FR); PHAM-HUU, Cuong, F-67700 Savernes (FR); GARIN, Francois, F-67300 Schiltigheim (FR); LEDOUX, Marc, F-67000 Strasbourg (FR); PHAM-HUU, Charlotte, F-67700 Savernes (FR)
(74) Mandataire: Pigasse, Daniel
(86) Numéro de dépôt international: PCT/FR2002/003697
(87) Numéro de publication internationale: WO 2003/037509

(56) Documents cités:
- DATABASE WPI Section Ch, Week 199832 Derwent Publications Ltd., London, GB; Class J04, AN 1998-371218 XP002213957 & JP 10 147771 A (TOTO LTD), 2 juin 1998 (1998-06-02)
- DATABASE WPI Section Ch, Week 199740 Derwent Publications Ltd., London, GB; Class D22, AN 1997-429732 XP002213958 & JP 09 192496 A (MATSUSHITA ELECTRIC WORKS LTD), 29 juillet 1997 (1997-07-29)
- DATABASE WPI Section Ch, Week 199825 Derwent Publications Ltd., London, GB; Class L01, AN 1998-280305 XP002213959 & JP 10 095635 A (TOTO LTD), 14 avril 1998 (1998-04-14)

## Description

### Domaine technique de l'invention

La présente invention concerne la purification, à température ambiante, d'effluents contenant des composés organiques volatils (COV) par l'intermédiaire d'un semi-conducteur composite WO₃ - SiC/TiO₂ soumis à une irradiation. Le photocatalyseur ainsi constitué est illuminé par une radiation, dont la longueur d'onde est au moins en partie inférieure à 400 nm. L'oxydation photocatalytique des polluants aboutit à une minéralisation totale en CO₂ et H₂O.

### Etat de la technique

Les composés organiques volatils (COV) sont utilisés ou produits dans de nombreuses activités industrielles et domestiques. De ce fait, on les trouve dans les sols, les eaux ainsi que dans l'atmosphère. Cela pose plusieurs problèmes d'environnement et de santé publique. D'abord, certains COV sont considérés comme une nuisance olfactive. Puis, une grande partie des COV est considérée comme étant cancérigène ou mutagène. Et finalement, l'émission des COV dans l'atmosphère est liée à la possible production photochimique d'oxydants à travers la réaction impliquant les COV et les NOₓ en présence de lumière. Ainsi, ces réactions mènent à une augmentation de l'ozone troposphérique, qui est toxique pour les humains, dégrade les récoltes et est impliqué dans la formation de pluies acides. Certains COV sont également impliqués dans la diminution de la couche d'ozone stratosphérique et sont susceptibles de contribuer au réchauffement global de la planète. Etant donné qu'un grand nombre de ces COV peut être oxydé, la catalyse hétérogène d'oxydation peut être utilisée pour les détruire dans les effluents. Mais, en général, ces catalyseurs opèrent à températures élevées, ce qui présente des inconvénients car il faut prévoir des dispositifs de chauffage et de régulation de température. Il serait plus simple de pouvoir conduire les réactions à la température ambiante, c'est-à-dire typiquement à une température comprise entre 15 et 30 °C.

Pour pouvoir détruire les COV à la température ambiante, l'oxydation photocatalytique de composés organiques en phase gaz, utilisant des catalyseurs à base de TiO₂, couplé ou non avec d'autres semiconducteurs, a été envisagée. A titre d'exemple, l'oxydation photocatalytique d'acétone avec un catalyseur de TiO₂ pur ou un catalyseur mixte TiO₂ / ZrO₂ à 77 °C, préparé sous forme de couche mince par un procédé sol-gel, a été décrite par M.E. Zorn et al. dans l'article « Photocatalytic oxidation of acetone vapor on TiO₂/ZrO₂ thin films », paru dans la revue Applied Catalysis B : Environmental, vol. 23, p. 1 - 8 (1999). Des photocatalyseurs de type TiO₂ / Pt ont été utilisés pour décomposer l'éthanol à une température de l'ordre de 200 °C (voir J.C. Kennedy et A.K. Datye, Journal of Catalysis, vol 179, p. 375 - 389 (1998). Un photocatalyseur mixte de type TiO₂ / CdS a été essayé pour la décomposition du phénol, du 2-chlorphénol et du pentachlorophénol en phase liquide (voir N. Serpone et al., « Exploiting the interparticle electron transfer process in the photocatalysed oxidation of phenol, 2-chlorophenol and pentachlorophenol : chemical evidence for electron and hole transfer between coupled semiconductors », Journal of Photchemistry and Photobiology A : Chemistry, vol 85, p. 247 - 255 (1995)). La demande de brevet allemand DE 40 23 995 A1 décrit des photocatalyseurs semiconducteurs à base d'oxyde de titane, titanate ou oxyde de zinc, éventuellement recouverts de métaux tels que Pt, Pd, Ir, Rh, Rn, Os, Zn ou Ba. Ce document propose l'utilisation de ces photocatalyseurs pour purifier l'air à l'intérieur de voitures.

Le principal intérêt de la photocatalyse est que l'énergie nécessaire pour les réactions d'oxydation est fournie par absorption directe de lumière plutôt que par chauffage thermique. Les photocatalyseurs utilisés à cette fin sont des semi-conducteurs ayant une bande interdite (gap optique) typiquement comprise entre environ 3 et environ 4 eV correspondant à une irradiation lumineuse dans la région spectrale du proche UV.

En général, les réactions photocatalytiques sur TiO₂ sont des réactions d'oxydoréduction et comportent plusieurs étapes principales :
■ Adsorption des réactifs, et notamment du polluant organique ;
■ Production de paires électrons - trous par absorption de photons issus de l'irradiation UV ;
■ Séparation spatiale des paires électrons - trous et migration à la surface du photocatalyseur ;
■ Réactions rédox des éléctrons et trous avec les espèces adsorbées en surface : réduction d'un accepteur d'électrons par l'électron, oxydation d'un donneur d'électrons par le trou ;
■ Désorption des produits de réaction.

Le plus souvent, l'accepteur d'électrons qui est réduit par l'électron est l'oxygène. Les trous peuvent se combiner directement avec les COV. Mais l'oxydation photocatalytique des COV peut aussi procéder par l'intermédiaire de radicaux, tels que OH et O.

La photocatalyse hétérogène utilisant des catalyseurs à base de TiO₂ possède plusieurs avantages :
i) le TiO₂ est relativement bon marché,
ii) il n'est pas nécessaire d'ajouter d'autres réactifs (autres que l'air et le COV),
iii) le procédé peut être conduit à température ambiante et pression atmosphérique,
iv) en général les produits de réactions se résument à CO₂ et H₂O.

Mais les photocatalyseurs à base de TiO₂ souffrent aussi de plusieurs inconvénients, exposés ci-dessous.

### Problème posé

L'activité du photocatalyseur selon l'état de la technique est limitée par le rendement du processus photocatalytique lui-même, ainsi que par l'adsorption trop importante de produits de réaction tel que le CO₂ ou d'éventuels produits intermédiaires d'oxydation qui peuvent bloquer une partie des sites actifs, ce qui conduit à la dégradation de l'activité du catalyseur.

Par ailleurs, l'utilisation d'un photocatalyseur selon l'état de la technique ne conduit pas toujours à une minéralisation complète des COV : Avec les photocatalyseurs connus à base de TiO₂, on observe, notamment lorsque la concentration en COV est élevée, la formation de produits d'oxydation partielle, dont certains sont toxiques.

Le problème auquel essaye de répondre la présente invention est donc de proposer un photocatalyseur à base de TiO₂ , à rendement amélioré et stable, pour la destruction par oxydation de composés organiques volatils dans les effluents gazeux.

### Objets de l'invention

La demanderesse a trouvé que l'utilisation d'une nouvelle formulation de photocatalyseur à base de semi-conducteurs composites de type WO₃ - SiC/TiO₂ permet d'améliorer les phénomènes d'adsorption de réactifs, de désorption des produits et de séparation spatiale des paires électrons - trous. Ainsi, l'activité photocatalytique se trouve augmentée et stabilisée par rapport aux photocatalyeurs à base de TiO₂ connus.

Un premier objet de la présente invention est constitué par un photocatalyseur à base d'au moins deux composés semiconducteurs couplés, caractérisé par le fait que l'un desdits semiconducteurs est le dioxyde de titane TiO₂, et l'autre est le carbure de silicium, SiC, et par le fait qu'il contient en plus du trioxyde de tungstène, WO₃.

Un deuxième objet de la présente invention est constitué par un procédé d'élaboration d'un photocatalyseur WO₃ - SiC / TiO₂ en deux étapes, une première étape de dépôt simultané du TiO₂ et du SiC sur un support, et une deuxième étape d'impregnation dudit dépôt par une solution comprenant au moins un précurseur de WO₃. Ledit précurseur est ensuite transformé en WO₃ par calcination.

Un troisième objet de la présente invention est constitué par l'utilisation dudit photocatalyseur dans un procédé de purification par oxydation photocatalytique d'effluents gazeux contenant des composés organiques volatils.

### Description des figures

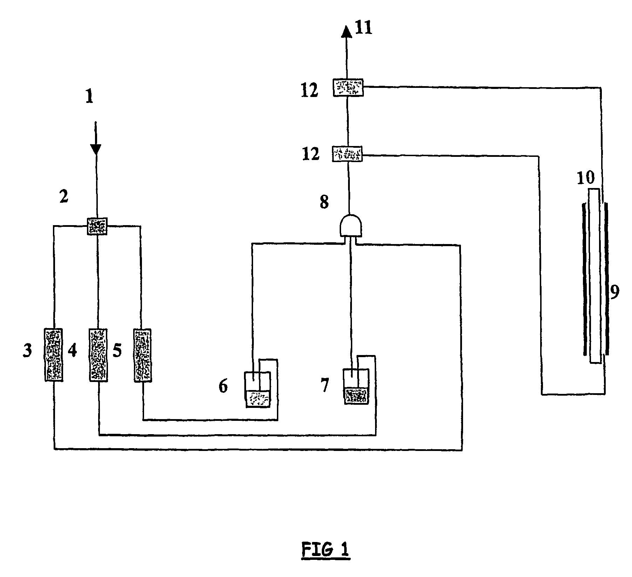

La figure 1 est un schéma du réacteur utilisé pour les essais. Les repères suivants sont utilisés :
- Arrivée principale d'air: 1
- Répartiteur: 2
- Voie air sec: 3
- Voie air humide: 4
- Voie COV: 5
- Saturateur contenant le polluant liquide: 6
- Bulleur contenant de l'eau liquide: 7
- Mélangeur: 8
- Réacteur photocatalytique: 9
- Lampe à rayons ultraviolets: 10
- Microchromatographe en phase gazeuse: 11
- Vannes: 12

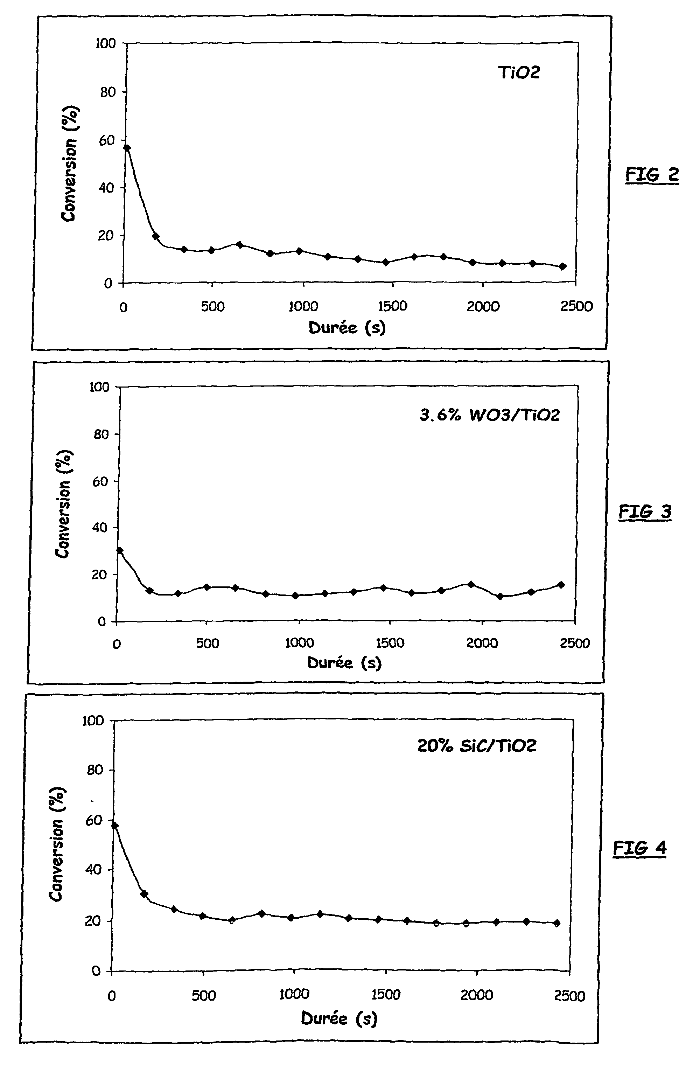

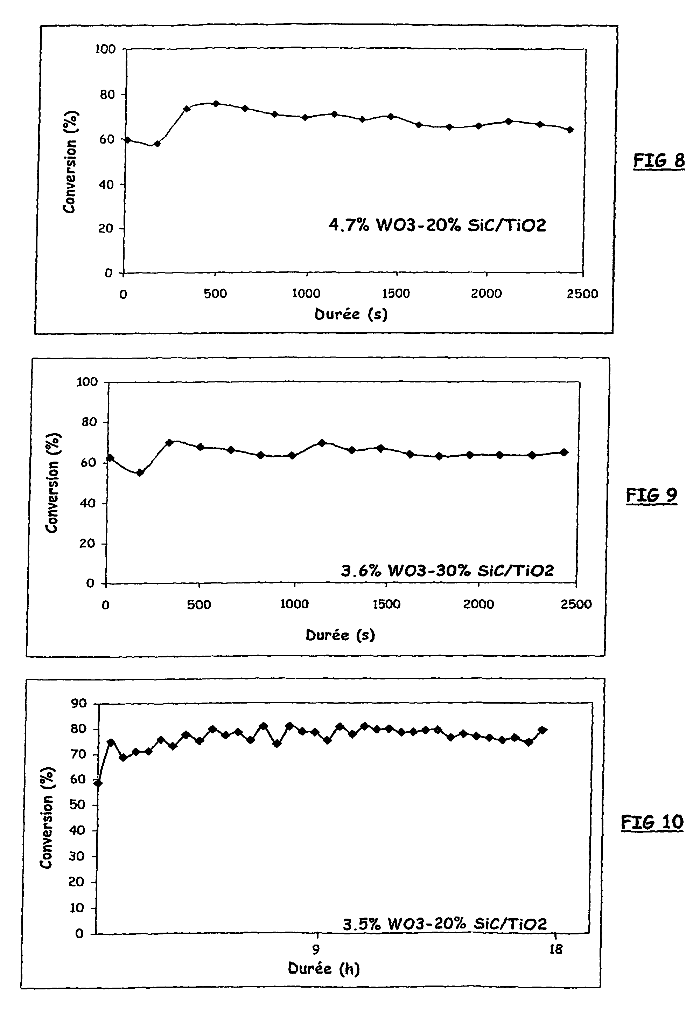

Les figures 2 à 10 sont relatives à des essais. Elles montrent en ordonnée le taux de conversion (en pourcent) du composé organique volatil choisi, et en abscisse la durée de l'essai (en secondes pour les figures 2 à 9, et en heures pour la figure 10), pour différentes formulations de photocatalyseurs selon l'invention ou selon l'état de la technique.

### Description détaillée de l'invention

Le photocatalyseur selon l'invention comprend au moins deux composés semiconducteurs couplés. Leur bande interdite (gap optique) se situe préférentiellement entre 3,0 et 3,2 eV. L'un desdit semiconducteurs est le dioxyde de titane, l'autre le SiC. La caractéristique essentielle du photocatalyseur selon l'invention est qu'il contient en plus du trioxyde de tungstène.

Dans une variante avantageuse de l'invention, le photocatalyseur selon l'invention contient entre 10 et 60 %, et préférentiellement entre 15 et 25 % de SiC (pourcentages massiques). Dans une autre variante avantageuse, il contient une quantité de WO₃ correspondant à 10 à 50 % d'une monocouche théorique sur les grains de TiO₂. Ledit TiO₂ a avantageusement une surface spécifique BET compnse entre 40 et 60 m² / g, et contient entre 1 et 5 %, et préférentiellement entre 1,5 et 4 % de WO₃ (pourcentages massiques).

Le photocatalyseur selon l'invention peut être préparé par un procédé en deux étapes : Procédé d'élaboration d'un photocatalyseur mixte WO₃- SiC/TiO₂, comprenant au moins une première étape au cours de laquelle on dépose simultanément le TiO₂ et le SiC sur un support, et une deuxième étape au cours de laquelle on impregne le dépôt de SiC / TiO₂ d'une solution d'un précurseur du WO₃. Ce procédé constitue le deuxième objet de la présente invention.

La première étape dudit procédé consiste à déposer simultanément le TiO₂ et le SiC. On prépare au cours cette première étape une première suspension à partir de poudre de TiO₂ et une deuxième suspension à partir d'une poudre de SiC, on mélange les deux suspensions, on les verse sur un support, et on fait évaporer le solvant. A titre d'exemple, comme poudre de TiO_{2,} une poudre commerciale avec une surface spécifique BET de l'ordre de 50 m²/g convient.

Le carbure de silicium peut être choisi parmi différents matériaux de SiC, et notamment ceux présentant une surface spécifique mesurée par BET comprise entre 1 et 600 m²/g.

La demanderesse a constaté qu'un SiC avec une surface spécifique entre 10 et 100 m²/g, et plus particulièrement entre 20 et 50 m²/g, donne de bons résultats. De tels matériaux peuvent être préparés par exemple selon les méthodes de synthèse décrites dans les brevets suivants : EP 0 313 480, EP 0 440 569, US 5,217,930, EP 0 511 919, EP 0 543 751 et EP 0 543 752. Selon ces méthodes, on peut synthétiser des matériaux de tailles et formes variées, c'est à dire sous forme de bâtonnets, de monolithes, d'extrudés, de grains ou de tubes. Ces types de SiC donnent de bons résultats, mais d'autres formes de SiC peuvent également être utilisées dans le cadre de la présente invention.

On prélève et mélange les volumes désirés de chacune des deux suspensions. Avantageusement, la quantité de TiO₂ déposée est choisie de façon à obtenir un recouvrement théorique de 1 mg/cm² sur le support. On verse la suspension ainsi préparée sur le support et répartit l'ensemble de façon homogène en chauffant jusqu'à évaporation complète de l'eau. Il est préférable de sècher à l'étuve à 120°C pendant 30 minutes. Dans un mode de réalisation préféré, le support est une paroi intérieure du réacteur dans lequel on utilise le photocatalyseur.

Au cours de la deuxième étape dudit procédé, on imprègne le dépôt de TiO₂ / SiC d'une solution comprenant au moins un précurseur du WO₃. Cela peut être fait avec une solution aqueuse du sel précurseur (NH₄)₁₀W₁₂O₄₁.5H₂O.

Dans un mode de réalisation avantageux de l'invention, le protocole d'imprégnation ainsi que le chauffage jusqu'à évaporation de la solution est le même que pour la première étape, y compris le séchage à l'étuve à 120°C pendant 30 minutes. Ensuite, on calcine sous air à une température comprise entre 300 et 500 °C, par exemple 420°C, pendant environ 1 h.

Le matériau du réacteur importe peu dans le cadre de la présente invention, à condition qu'il soit inerte. On peut utiliser par exemple des tubes en polypropylène, en fibre de carbone ou en fibre de verre, ou encore du verre ou du quartz. L'utilisation d'un matériau transparent au rayonnement ultraviolet (le quartz par exemple) est avantageuse lorsque la source de rayonnement UV se trouve à l'extérieur du réacteur, ou lorsque l'on utilise la lumière du soleil. Mais on peut aussi envisager un autre moyen pour faire passer le rayonnement, tel qu'une fenêtre en matériau transparent aux rayons ultraviolets.

Le photocatalyseur selon l'invention peut être utilisé dans un procédé de purification par oxydation photocatalytique d'effluents contenant des composés organiques volatils . Ce procédé comprend :
a) l'introduction des effluents gazeux dans un réacteur contenant un photocatalyseur selon l'invention ;
b) l'irradiation dudit photocatalyseur avec un rayonnement dont au moins une partie de la puissance lumineuse est émise avec une longueur d'onde inférieure à 400 nm et préférentiellement inférieure à 360 nm, de façon à ce qu'au moins une partie des composés organiques volatiles contenus dans lesdits effluents est décomposée par oxydation ;
c) la sortie du mélangé réactionnel gazeux du réacteur.

L'oxydation photocatalytique des composés organiques volatils (COV) est effectuée avantageusement comme un procédé continu à température ambiante, et à pression atmosphérique. Cela permet d'utiliser le photocatalyseur selon l'invention directement avec des effluents, par exemple industriels, agricoles ou domestiques, sans aucun prétaitement particulier.

Selon un mode de réalisation particulier, le procédé selon l'invention comprend l'ajout d'oxygène et / ou de la vapeur d'eau avant leur introduction dans le réacteur, ou l'introduction simultanée d'oxygène et / ou de vapeur d'eau dans le réacteur.

Les effluents introduits dans le réacteur peuvent être issus directement de procédés industriels, agricoles ou domestiques, ou résulter d'un prétraitement de tels effluents. Selon un mode de réalisation préféré du procédé selon l'invention, on introduit des effluents industriels, agricoles ou domestiques qui contiennent déjà une teneur suffisante d'oxygène et de vapeur d'eau, sans aucun ajout.

Un dispositif pour la purification d'effluents contenant des composés organiques volatils utilisable dans le cadre de la présente invention comprend au moins :
■ un réacteur comprenant le photocatalyseur selon l'invention ;
■ une source de rayonnement ultraviolet ;
■ un moyen d'adduction de l'effluent gazeux à purifier ;
■ un moyen d'évacuation des produits réactionnels.
La source de rayonnement UV se présente avantageusement sous forme d'une ou plusieurs lampes UV tubulaires émettant un rayonnement caractérisé par le fait qu'au moins une partie de sa puissance lumineuse est émise avec une longueur d'onde inférieure à 400 nm et préférentiellement inférieure à 360 nm.

Dans un mode de réalisation préféré, le photocatalyseur est déposé sur la paroi éclairée du réacteur photocatalytique, et la lampe UV est disposée à l'intérieur du réacteur photocatalytique. L'effluent gazeux circule de façon tangentielle entre la paroi externe des tubes UV et interne du réacteur. L'intervalle entre ces deux parois est ajusté de façon à optimiser le contact entre le flux gazeux et la surface du catalyseur tout en minimisant la perte de charge.

Un réacteur de type annulaire, coaxial, avec la lampe UV disposée à l'intérieur, convient à la réalisation de la présente invention, mais ce mode de réalisation ne limite pas la présente invention. Différentes géométries et configurations de réacteurs peuvent être envisagées. De même, le photocatalyseur selon l'invention peut être déposé sur différents supports.

Dans un autre mode de réalisation de l'invention, la puissance lumineuse est au moins en partie fournie par le rayonnement du soleil. Dans ce cas, le réacteur peut alors ne pas disposer d'une source technique de rayonnement UV, telle qu'une lampe appropriée, la source du rayonnement UV étant le soleil. Dans ce cas (ainsi que dans le cas ou la source technique de rayonemment UV se situe à l'extérieure du réacteur), le réacteur photocatalytique doit être pourvu d'un moyen pour faire passer ce rayonnement solaire ; ce moyen peut être une fenêtre en matériau transparent approprié qui laisse passer la lumière du soleil, ou le réacteur peut être construit en utilisant un tel matériau transparent approprié. Lorsque l'on utilise la lumière du soleil, cette lumière peut être concentrée et / ou focalisée à l'aide de dispositifs optiques connus de l'homme du métier.

Selon les constatations de la demanderesse, pour assurer une décomposition optimale d'effluents contenant des composés organiques volatils, il est avantageux d'ajuster la composition chimique du photocatalyseur en fonction de la nature, et notamment en fonction de sa polarité, de la principale molécule organique que l'on veut détruire, ainsi qu'en fonction de la surface spécifique du TiO₂ utilisé. Pour la plupart des COV, tels que la méthyléthylcétone, une teneur en WO₃ comprise entre 1,0 % et 5,0 % (pourcentages massiques) convient. Cette teneur optimale peut être déterminée par l'homme du métier à l'aide d'une simple expérimentation de routine ; un TiO₂ avec une grande surface spécifique nécessitera une teneur en WO₃ plus élevée qu'un TiO₂ avec une faible surface spécifique. Une teneur trop élevée en WO₃ risque de masquer le TiO₂, ce qui diminue l'efficacité du catalyseur, car une partie de la lumière est absorbée par le WO₃.

A titre d'exemple, pour un TiO₂ avec une surface spécifique BET de 50 m² / g, étant donné que pour WO₃, la monocouche théorique correspond en moyenne à 0,21 g / 100 m² de support (voir Y.C. Xie, Y.Q. Tang, dans Advances in Catalysis, vol. 37, page 1 (1990)), la teneur optimale en WO₃ comprise entre 0,1 % et 5,0 % correspond donc à environ 10 à 50 % de la monocouche théorique.

La teneur optimale en SiC, qui est toutefois moins critique, et le type de SiC utilisé au départ peuvent également être déterminés à l'aide d'une simple expérimentation de routine.

Le procédé peut être utilisé pour la purification d'effluents industriels, agricoles ou domestiques. A titre d'exemple, des effluents gazeux issus d'une tannerie, qui représentent une nuisance olfactive certaine, ont été traités avec succès avec le procédé selon l'invention. Dans ces effluents, les principaux COV étaient la méthyléthylcétone et l'acétate de butyle. Ce traitement était effectué à la température ambiante (c'est-à-dire une température typiquement comprise entre 15 et 30 °C) et à pression ambiante (c'est-à-dire à la pression atmosphérique), ce qui est avantageux car le réacteur peut ainsi être d'une conception très simple et robuste. Un débit d'effluents gazeux industriels jusqu'à 10 000 m³/h a ainsi été traité avec succès dans un réacteur prototype. La demanderesse a également constaté que le photocatalyseur selon l'invention permet la désinfection du flux gazeux qui le traverse ; par conséquent, il peut être utilisé pour inactiver des micro-organismes, tels que des bactéries ou des virus, contenus dans l'air.

L'invention sera mieux comprise à l'aide des exemples, qui n'ont toutefois pas de caractère limitatif.

### Exemples

Dans ces exemples, tous les pourcentages qui concernent la composition chimique du photocatalyseur sont des pourcentages massiques.

Un schéma du réacteur utilisé pour les essais est montré sur la figure 1. Le réacteur photocatalytique (9) était de type annulaire. La source de rayonnement ultraviolet (10) était une lampe tubulaire montée de manière coaxiale à l'intérieur du réacteur; la longueur d'onde du rayonnement était centrée sur 350 à 360 nm.

L'arrivée principale d'air (1) se divise en trois voies grâce à un répartiteur (2). Le débit de chacune des trois voies, air sec (3), air humide (4) et COV (5), est fixé par l'intermédiaire d'un débimètre massique. L'air est saturé en vapeur d'eau et en COV en traversant un bulleur contenant de l'eau liquide (7) et en COV grâce à un saturateur contenant le polluant liquide (6). Après avoir fixé les débits sur chaque voie et par conséquent les concentrations en COV et le taux d'humidité relatif, l'ensemble passe au travers d'un mélangeur (8). Grâce aux vannes (12), le mélange réactionnel peut ensuite être orienté soit sur le catalyseur (9) irradié par le rayonnement UV (10), suivi d'un microchromatographe en phase gaz (11) pour analyse des produits de réaction, soit directement sur le microchromatographe pour analyse de la composition gazeuse initiale.

Différentes formulations du catalyseur WO₃ - SiC/TiO₂ ont été testées, en faisant varier leurs compositions relatives en WO₃ et en SiC. Le SiC a été extrudé, lavé, étuvé, calciné à 700°C puis broyé. Le TiO₂ utilisé était une poudre commerciale (fournisseur : société Prolabo) et le SiC a été obtenu selon le procédé décrit dans la demande de brevet EP 0 543 751 A1, dont l'invention permet de synthétiser des carbures métalliques et de silicium de haute surface spécifique en faisant réagir un oxyde volatil du silicium, SiO avec du carbone à haute surface spécifique à une température comprise entre 900 et 1400°C sous balayage d'un gaz inerte.

Différentes expériences, référencées 1, 2, 3, 4, 5, 6, 7, 8 et 9 ont été menées, toutes pour des teneurs en humidité de 50%. Le composé organique volatil de référence a été la méthyléthylcétone (MEK) à raison de 1500 ppm (ppm massiques). Les essais ont été effectués sous mélange réactionnel continu pour des durées comprises entre 40 min et 18 heures.

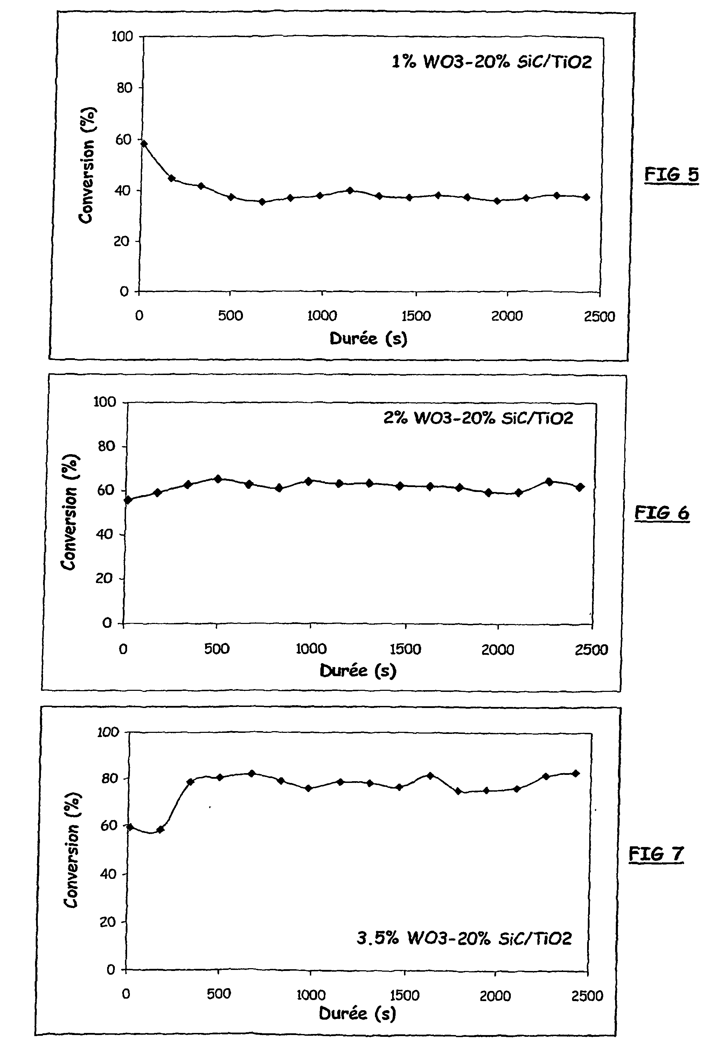

Les expériences 1, 2, 3 et 4 sont relatives à l'influence de la nature des différents matériaux, TiO₂, WO₃ et SiC.
L'expérience 1 a été effectuée sur le catalyseur TiO₂ seul (figure 2).
L'expérience 2 a été effectuée sur la catalyseur 3.6% WO₃/TiO₂ (figure 3).
L'expérience 3 a été effectuée sur le catalyseur 20% SiC/TiO₂ (figure 4)
L'expérience 4 a été effectuée sur le catalyseur 1% WO₃ - 20% SiC/TiO₂ (figure 5).

Les expériences 5, 6 et 7 étudient l'influence de la teneur en oxyde de tungstène.
L'expérience 5 a été effectuée sur le catalyseur 2% WO₃ - 20% SiC/TiO₂ (figure 6).
L'expérience 6 a été réalisée sur le catalyseur 3.5% WO₃ - 20% SiC/TiO₂ (figure 7).
L'expérience 7 a été réalisée sur le catalyseur 4.7% WO₃ -20% SiC/TiO₂ (figure 8)

L'expérience 8 est relative à l'influence de la teneur en SiC. Elle a été réalisée sur le catalyseur 3.6% WO₃ -30% SiC/TiO₂ (figure 9).
L'expérience 9 représente une étude de stabilité du catalyseur composite sous mélange réactionnel. Elle a été réalisée sur le catalyseur 3.5% WO₃ - 20% SiC/TiO₂ (figure 10) après plus de 18 heures sous mélange réactionnel.

Bien que la conversion initiale soit d'environ 60% sur le TiO₂ seul (figure 2), ce catalyseur n'est pas très efficace pour la destruction du polluant, puisqu'il se désactive assez rapidement en fonction du temps sous mélange réactionnel, pour arriver à des taux de conversion se situant aux alentours de 10 %. L'ajout de 3.6% de WO₃ au TiO₂ n'améliore pas le comportement photocatalytique du système (figure 3). L'utilisation du catalyseur semi-conducteur composite, 20% SiC/TiO₂, conduit à une légère amélioration par rapport au TiO₂ seul (expérience 3, figure 4) puisqu'on arrive à des taux de conversion d'environ 20% après environ 1000 à 2000 secondes. Par contre, l'addition de WO₃ et de SiC à TiO₂ (figures 5, 6, 7, 8, et 9) résulte en une augmentation considérable des propriétés de photooxydation du COV, dépendant de la teneur en WO₃ et en SiC. On constate que le pourcentage massique de SiC optimal est de 20% et celui de WO₃ est de 3.5%. Ainsi, la formulation optimale du photocatalyseur pour decomposer la méthyléthylcétone est 3.5% WO₃ - 20% SiC/TiO₂. Dans ces conditions, après trois minutes de mise en régime, on transforme 80% du polluant en CO₂ et H₂O. En augmentant les proportions relatives de SiC et de WO₃, on diminue cette activité. L'expérience 9 (figure 10) représente le comportement du catalyseur 3.5% WO₃ - 20% SiC/TiO₂ sous flux continu de méthyléthylcétone pendant plus de 18 heures. On observe qu'il n'y a aucune désactivation du catalyseur, ce qui correspond à une importante amélioration par rapport à l'état de la technique.

Des essais complémentaires, effectués dans les mêmes conditions que les précédents, ont permis de montrer qu'un photocatalyseur avec 50 % de SiC et 3,4 % de WO₃ donne un taux de conversion sur environ 2 000 secondes de l'ordre de 40 %, et un photocatalyseur avec 8 % de SiC et 3,5 % de WO₃ donne un résultat sensiblement égal à celui observé lors de l'expérience 3.

## Revendications

1. Photocatalyseur à base d'au moins deux composés semiconducteurs couplés, **caractérisé par le fait que** l'un desdits semiconducteurs est le dioxyde de titane TiO₂, et l'autre est le carbure de silicium, SiC, et **par le fait qu'**il contient en plus du trioxyde de tungstène, WO₃.

2. Photocatalyseur selon la revendication 1, **caractérisé en ce qu'**il contient entre 10 et 60 %, et préférentiellement entre 15 et 25 % de SiC (pourcentages massiques),

3. Photocatalyseur selon une quelconque des revendication 1 ou 2, **caractérisé en ce qu'**il contient une quantité de WO₃ correspondant à 10 à 50 % d'une monocouche théorique par rapport à la surface spécifique BET des grains de TiO₂.

4. Photocatalyseur selon une quelconque des revendications 1 à 3, **caractérisé en ce qu'**il contient du TiO₂ avec une surface spécifique BET comprise entre 40 et 60 m² / g, et **en ce qu'**il contient entre 1 et 5 %, et préférentiellement entre 1,5 et 4 % de WO₃ (pourcentages massiques).

5. Photocatalyseur selon une quelconque des revendications 1 à 4, **caractérisé en ce qu'**il contient du SiC avec une surface spécifique BET comprise entre 1 et 600 m² / g et préferentiellement entre 10 et 100 m² / g.

6. Procédé d'élaboration d'un photocatalyseur mixte WO₃- SiC/TiO₂, comprenant au moins une première étape au cours de laquelle on dépose simultanément le TiO₂ et le SiC sur un support, et une deuxième étape au cours de laquelle on impregne le dépôt de SiC / TiO₂ d'une solution contenant au moins un précurseur du WO₃.

7. Procédé selon la revendication 6, **caractérisé en ce que** l'on prépare au cours de la première étape une première suspension à partir de poudre de TiO₂ et une deuxième suspension à partir d'une poudre de SiC, on mélange les deux suspensions, on les verse sur un support, et on fait évaporer le solvant.

8. Procédé selon une quelconque des revendications 6 et 7, **caractérisé en ce que** la solution du précurseur de WO₃ est une solution aqueuse de (NH₄)₁₀W₁₂O₄₁.5H₂O.

9. Procédé selon une quelconque des revendications 6 à 8, **caractérisé en ce qu'**il comprend en plus une troisième étape, au cours de la quelle on effectue une calcination à une température préférentiellement comprise entre 300 et 500 °C.

10. Utilisation d'un photocatalyseur selon une quelconque des revendications 1 à 5 dans un procédé de purification d'effluents contenant des composés organiques volatiles par oxydation photocatalytique, ledit procédé comprenant :
a) l'introduction des effluents dans un réacteur contenant ledit photocatalyseur ;
b) l'irradiation du photocatalyseur par un rayonnement dont au moins une partie de la puissance lumineuse est émise avec une longueur d'onde inférieure à 400 nm et préférentiellement inférieure à 360 nm, de façon à ce qu'au moins une partie des composés organiques volatils contenus dans lesdits effluents soit décomposée par oxydation ;
c) la sortie du mélangé réactionnel gazeux du réacteur.

11. Utilisation d'un photocatalyseur selon une quelconque des revendications 1 à 5 dans un procédé d'inactivation de micro-organismes contenus dans un flux gazeux par oxydation photocatalytique, ledit procédé comprenant :
a) l'introduction d'un flux gazeux dans un réacteur contenant ledit photocatalyseur ;
c) l'irradiation du photocatalyseur par un rayonnement dont au moins une partie de la puissance lumineuse est émise avec une longueur d'onde inférieure à 400 nm et préférentiellement inférieure à 360 nm, de façon à ce qu'au moins une partie des micro-organismes contenus dans ledit flux gazeux soit décomposée par oxydation ;
d) la sortie du flux gazeux du réacteur.

12. Utilisation selon une des revendications 10 ou 11, **caractérisée en ce que** ledit procédé est conduit à une température comprise entre 15 et 30 °C.

13. Utilisation selon une quelconque des revendications 10 à 12, **caractérisée en ce que** ledit procédé est conduit à la pression atmosphérique.

14. Utilisation selon une quelconque des revendications 10 à 13, **caractérisée en ce que** ladite puissance lumineuse provient au moins en partie du rayonnement solaire.

## Patentansprüche

1. Photokatalysator auf Basis von mindestens zwei gekoppelten Halbleiterverbindungen, **dadurch gekennzeichnet, dass** einer der Halbleiter Titandioxid TiO₂ und der andere Siliciumcarbid SiC ist und dass er zusätzlich Wolframtrioxid WO₃ enthält.

2. Photokatalysator nach Anspruch 1, **dadurch gekennzeichnet, dass** er 10 bis 60 % und vorzugsweise 15 bis 25 % SiC (Massenprozente) enthalt.

3. Photokatalysator nach irgendeinem der Anspruche 1 oder 2, **dadurch gekennzeichnet, dass** er eine WO₃-Menge enthalt, die 10 bis 50 % einer einlagigen Sollschicht bezogen auf die spezifische Oberflache BET der TiO₂-Körner entspricht.

4. Photokatalysator nach irgendeinem der Ansprüche 1 bis 3, **dadurch gekennzeichnet, dass** er TiO₂ mit einer spezifischen Oberflache BET von 40 bis 60 m²/g enthält und dass er 1 bis 5 % und vorzugsweise 1,5 bis 4 % WO₃ (Massenprozente) enthalt.

5. Photokatalysator nach irgendeinem der Ansprüche 1 bis 4, **dadurch gekennzeichnet, dass** er SiC mit einer spezifischen Oberflache BET von 1 bis 600 m²/g und vorzugsweise 10 bis 100 m²/g enthalt.

6. Verfahren zur Herstellung eines Photokatalysators zusammengesetzt aus WO₃ und SiC/TiO_{2,} umfassend mindestens einen ersten Schritt, bei dem TiO₂ und SiC gleichzeitig auf ein Tragermaterial abgeschieden werden, und einen zweiten Schritt, bei dem die SiC/TiO₂-Abscheidung mit einer Lösung getränkt wird, die mindestens ein Vorprodukt für WO₃ enthält.

7. Verfahren nach Anspruch 6, **dadurch gekennzeichnet, dass** beim ersten Schritt eine erste Suspension aus TiO₂-Pulver und eine zweite Suspension aus einem SiC-Pulver hergestellt wird, die beiden Suspensionen gemischt werden, auf ein Tragermaterial gegossen werden und das Losungsmittel ausgedampft wird.

8. Verfahren nach irgendeinem der Ansprüche 6 und 7, **dadurch gekennzeichnet, dass** die Lösung des WO₃-Vorprodukts eine wassrige Losung aus (NH₄)₁₀W₁₂O₄₁ 5H₂O ist.

9. Verfahren nach irgendeinem der Anspruche 6 bis 8, **dadurch gekennzeichnet, dass** es einen dritten Schritt umfasst, bei dem bei einer Temperatur von vorzugsweise 300 bis 500 °C eine Calcinierung durchgeführt wird.

10. Verwendung eines Photokatalysators nach irgendeinem der Ansprüche 1 bis 5 in einem Verfahren zur Reinigung von fluchtige organische Verbindungen enthaltenden Stoffen durch photokatalytische Oxidation, welches Verfahren umfasst:
a) die Einleitung der Stoffe in einen den Photokatalysator enthaltenden Reaktor;
b) die Bestrahlung des Photokatalysators mit einer Strahlung, bei der zumindest ein Teil der Lichtleistung mit einer Wellenlänge kleiner als 400 nm und vorzugsweise kleiner als 360 nm emittiert wird, so dass zumindest ein Teil der in den Stoffen enthaltenen flüchtigen organischen Verbindungen durch Oxidation abgebaut wird;
c) die Ableitung des gasformigen Reaktionsgemischs aus dem Reaktor.

11. Verwendung eines Photokatalysators nach irgendeinem der Ansprüche 1 bis 5 in einem Verfahren zur Inaktivierung von in einem Gasstrom enthaltenen Mikroorganismen durch photokatalytische Oxidation, welches Verfahren umfasst.
a) die Einleitung eines Gasstroms in einen den Photokatalysator enthaltenden Reaktor,
b) die Bestrahlung des Photokatalysators mit einer Strahlung, bei der zumindest ein Teil der Lichtleistung mit einer Wellenlänge kleiner als 400 nm und vorzugsweise kleiner als 360 nm emittiert wird, so dass zumindest ein Teil der im Gasstrom enthaltenen Mikroorganismen durch Oxidation abgebaut wird;
c) die Ableitung des Gasstroms aus dem Reaktor.

12. Verwendung nach einem der Anspruche 10 oder 11, **dadurch gekennzeichnet, dass** das Verfahren bei einer Temperatur von 15 bis 30 °C durchgefuhrt wird.

13. Verwendung nach irgendeinem der Ansprüche 10 bis 12, **dadurch gekennzeichnet, dass** das Verfahren bei atmosphärischem Druck durchgeführt wird.

14. Verwendung nach irgendeinem der Anspruche 10 bis 13, **dadurch gekennzeichnet, dass** die Lichtleistung zumindest teilweise aus der Sonnenstrahlung stammt.

## Claims

1. Photocatalyst containing at least two coupled semiconductor compounds, **characterized in that** one of said semiconductors is titanium dioxide TiO₂, and the other is silicon carbide, SiC, and **in that** it also contains tungsten trioxide, WO₃.

2. Photocatalyst according to claim 1, **characterized in that** it contains between 10 and 60 %, preferably between 15 and 25 % SiC (weight percentages).

3. Photocatalyst according to either of claims 1 or 2, **characterized in that** it contains a quantity of WO₃ corresponding to 10 to 50 % of a theoretical monolayer relative to the BET specific surface area of TiO₂ particles.

4. Photocatalyst according to any of claims 1 to 3, **characterized in that** it contains TiO₂ having a BET specific surface area of between 40 and 60 m²/g, and **in that** it contains between 1 and 5 %, preferably between 1.5 and 4 % WO₃ (weight percentages).

5. Photocatalyst according to any of claims 1 to 4, **characterized in that** it contains SiC having a BET specific area of between 1 and 600 m²/g, preferably between 10 and 100 m²/g.

6. Process for preparing a mixed WO₃-SiC/TiO₂ photocatalyst, comprising at least a first step during which the TiO₂ and SiC are simultaneously deposited on a carrier, and a second step during which the SiC/TiO₂ deposit is impregnated with a solution containing at least one WO₃ precursor.

7. Process according to claim 6, **characterized in that** during the first step a first suspension is prepared from TiO₂ powder and a second suspension from SiC powder, the two suspensions are mixed, poured onto a carrier and the solvent is evaporated.

8. Process according to either of claims 6 and 7, **characterized in that** the WO₃ precursor solution is an aqueous solution of (NH₄)₁₀W₁₂O₄₁.5H₂O.

9. Process according to any of claims 6 to 8, **characterized in that** it also comprises a third step, during which calcination is conducted at a temperature of preferably between 300 and 500°C.

10. Use of a photocatalyst according to any of claims 1 to 5, in a process for purifying effluent containing volatile organic compounds by photocatalytic oxidation, said process comprising:
a) adding the effluent to a reactor containing said photocatalyst,
b) irradiating the photocatalyst with radiation of which at least part of the light power is emitted along a wavelength of less than 400 nm and preferably less than 360 nm, so that at least part of the volatile organic compounds contained in said effluent is decomposed by oxidation;
c) evacuating the gaseous reaction mixture from the reactor.

11. Use of a photocatalyst according to any of claims 1 to 5 in a process for inactivating microorganisms contained in a gaseous flow by photocatalytic oxidation, said process comprising:
a) adding a gaseous flow to a reactor containing said photocatalyst,
b) irradiating the photocatalyst by radiation of which at least part of the light power is emitted along a wavelength of less than 400 nm and preferably less than 360 nm, so that at least part of the microorganisms contained in said gas flow are decomposed by oxidation,
d) evacuating the gas flow from the reactor.

12. Use according to either of claims 10 or 11, **characterized in that** said process is conducted at a temperature of between 15 and 30 °C.

13. Use according to any of claims 10 to 12, **characterized in that** said process is conducted at atmospheric pressure.

14. Use according to any of claims 10 to 13, **characterized in that** said light power is derived at least in part from sun radiation.
